# EUROPEAN PATENT APPLICATION

(11) **EP 4 483 898 A1**
(43) Date of publication of application: **01.01.2025**
(21) Application number: 23199080.5
(22) Date of filing: 22.09.2023
(51) Int. Cl.: A61K 39/12, A61P 31/14, A61P 31/16

(54) **INTRANASAL VACCINE COMPOSITION AND METHOD FOR BOOSTING USING THE SAME**

(30) Priority: 28.06.2023 US 202363523876 P
(71) Applicant: Advagene Biopharma Co., Ltd., Taipei City (TW)
(72) Inventor: HSU, Yu-Shen, Taipei City (TW); KANG, Ssu-Wei, Taipei City (TW); CHANG, Mingi, Taipei City (TW)
(74) Representative: Vossius & Partner Patentanwälte Rechtsanwälte mbB

(57) **Abstract**

The present disclosure provides a method for vaccinating a subject against a mucosal virus infection, comprising administering to the subject an immunologically effective amount of an intranasal booster, wherein the intranasal booster comprises detoxified *Escherichia coli* labile toxin (LT) and an antigen from the mucosal virus, and wherein the subject has been previously primed. An intranasal vaccine composition comprising an immunologically effective amount of a mucosal virus antigen adjuvanted with a detoxified *Escherichia coli* labile toxin (LT) is also provided.

## Description

### Field of the Invention

The invention relates an intranasal vaccine composition comprising a mucosal virus antigen adjuvanted with a detoxified *Escherichia coli* labile toxin (LT), and a method to prevent mucosal virus infection using the same. Particularly, the intranasal vaccine composition is a booster that prevents coronavirus or influenza virus infection.

### Background of the Invention

In late December 2019, the first case of SARS-CoV-2 was identified in Wuhan, China. This virus caused a global pandemic. Several novel vaccines for SARS-CoV-2 of different mechanisms have since entered phase III trial or received EUA approval. The vaccines include nucleoside-modified mRNA vaccines, adeno-based vaccines, and recombinant subunit vaccines. The rapid development of vaccines for SARS-CoV-2 represents an unprecedented achievement for biotech. However, the SARS-CoV-2 variants, such as Omicron, exhibit increased immune evasion, reducing vaccine effectiveness (Cohn BA, Cirillo PM, Murphy CC, Krigbaum NY, Wallace AW: SARS-CoV-2 vaccine protection and deaths among US veterans during 2021. Science 2022, 375(6578):331-336; Lucas C, Vogels CBF, Yildirim I, Rothman JE, Lu P, Monteiro V, Gehlhausen JR, Campbell M, Silva J, Tabachnikova A et al: Impact of circulating SARS-CoV-2 variants on mRNA vaccine-induced immunity. Nature 2021, 600(7889):523-529; Schmidt F, Muecksch F, Weisblum Y, Da Silva J, Bednarski E, Cho A, Wang Z, Gaebler C, Caskey M, Nussenzweig MC et al: Plasma neutralization of the SARS-CoV-2 omicron variant. N Engl J Med 2022, 386(6):599-601). At present, except for one adenovector-based intranasal vaccine by Bharat (iNCOVACC), all other SARS-CoV-2 vaccines require intramuscular (IM) administration. Vaccines via the IM route have a track record of good safety and providing robust humoral immunity; however, there is often diminished efficacy four months after the IM booster (Cohn BA, Cirillo PM, Murphy CC, Krigbaum NY, Wallace AW: SARS-CoV-2 vaccine protection and deaths among US veterans during 2021. Science 2022, 375(6578):331-336; Cele S, Jackson L, Khoury DS, Khan K, Moyo-Gwete T, Tegally H, San JE, Cromer D, Scheepers C, Amoako DG et al: Omicron extensively but incompletely escapes Pfizer BNT162b2 neutralization. Nature 2022, 602(7898):654-656; Chemaitelly H, Tang P, Hasan MR, AlMukdad S, Yassine HM, Benslimane FM, Al Khatib HA, Coyle P, Ayoub HH, Al Kanaani Z et al: Waning of BNT162b2 Vaccine Protection against SARS-CoV-2 Infection in Qatar. N Engl J Med 2021, 385(24):e83). Another critical issue with IM vaccination is poor mucosal immunity, which is likely essential to disrupt SARS-CoV-2 infection and prevent transmission (Mouro V, Fischer A: Dealing with a mucosal viral pandemic: lessons from COVID-19 vaccines. Mucosal Immunol 2022, 15(4):584-594; Li M, Wang H, Tian L, Pang Z, Yang Q, Huang T, Fan J, Song L, Tong Y, Fan H: COVID-19 vaccine development: milestones, lessons and prospects. Signal Transduct Target Ther 2022, 7(1):146; Russell MW, Moldoveanu Z, Ogra PL, Mestecky J: Mucosal Immunity in COVID-19: A Neglected but Critical Aspect of SARS-CoV-2 Infection. Front Immunol 2020, 11:611337; Russell MW, Mestecky J: Mucosal immunity: The missing link in comprehending SARS-CoV-2 infection and transmission. Front Immunol 2022, 13:957107; Huang M, Zhang M, Zhu H, Du X, Wang J: Mucosal vaccine delivery: A focus on the breakthrough of specific barriers. Acta Pharm Sin B 2022, 12(9):3456-3474).

Mucosal immunity is a compartmentalized immune response playing a critical role in defending against infection via mucosa. Mucosal vaccination elevates infection-specific mucosal immunity to infecting sites (Miquel-Clopes A, Bentley EG, Stewart JP, Carding SR: Mucosal vaccines and technology. Clin Exp Immunol 2019, 196(2):205-214). The secretory IgA (sIgA) is one marker of mucosal immunity. Unlike the effect of IgG in protecting against progression of disease symptoms, sIgA responds to infectious microbes at infected sites. Regardless of the critical role of mucosal immunity against SARS-CoV-2, only one mucosal vaccine developed by Bharat India has been approved (Waltz E: China and India approve nasal COVID vaccines-are they a game changer? Nature 2022, 609(7927):450). The delay in the development of mucosal vaccines may be due to the tolerogenic nature of epithelium track and the concerns over neurological adverse events induced by intranasal adjuvants.

The current vaccines for COVID-19 comprise the IM administration of SARS-CoV-2 coding nucleotides or recombinant spike antigen (S-2P) to induce cellular and humoral immunity. Although IM vaccines induce robust spike-specific IgG, clinical evidence has shown that priming and boosting via IM routes are insufficient to cease SARS-CoV-2 transmission. To overcome the deficiency, there is desperate need for an effective mucosal vaccine to terminate the infection by and transmission of the virus.

*Escherichia coli* heat-labile toxin (LT) has been extensively studied as an effective intranasal (IN) adjuvant for over 30 years (Clements JD, Hartzog NM, Lyon FL: Adjuvant activity of Escherichia coli heat-labile enterotoxin and effect on the induction of oral tolerance in mice to unrelated protein antigens. Vaccine 1988, 6(3):269-277; Hashigucci K, Ogawa H, Ishidate T, Yamashita R, Kamiya H, Watanabe K, Hattori N, Sato T, Suzuki Y, Nagamine T et al: Antibody responses in volunteers induced by nasal influenza vaccine combined with Escherichia coli heat-labile enterotoxin B subunit containing a trace amount of the holotoxin. Vaccine 1996, 14(2):113-119; Stephenson I, Zambon MC, Rudin A, Colegate A, Podda A, Bugarini R, Del Giudice G, Minutello A, Bonnington S, Holmgren J et al: Phase I evaluation of intranasal trivalent inactivated influenza vaccine with nontoxigenic Escherichia coli enterotoxin and novel biovector as mucosal adjuvants, using adult volunteers. J Virol 2006, 80(10):4962-4970; Walker RI: New strategies for using mucosal vaccination to achieve more effective immunization. Vaccine 1994, 12(5):387-400). LTh(αK) is a fully detoxified *Escherichia coli* LT protein from human isolate. In two IN influenza vaccine clinical trials, LTh(αK)-adjuvanted inactivated intranasal vaccine (IIV) elicited both serum IgG and sIgA against hemagglutination (Pan SC, Hsieh SM, Lin CF, Hsu YS, Chang M, Chang SC: A randomized, double-blind, controlled clinical trial to evaluate the safety and immunogenicity of an intranasally administered trivalent inactivated influenza vaccine with adjuvant LTh(alphaK): A phase I study. Vaccine 2019, 37(14):1994-2003; Pan SC, Hsu WT, Lee WS, Wang NC, Chen TJ, Liu MC, Pai HC, Hsu YS, Chang M, Hsieh SM: A double-blind, randomized controlled trial to evaluate the safety and immunogenicity of an intranasally administered trivalent inactivated influenza vaccine with the adjuvant LTh(alphaK): A phase II study. Vaccine 2020, 38(5):1048-1056). However, there was no report of the LTh(αK)-adjuvanted inactivated intranasal vaccine (IIV) being applied to the SARS-CoV-2 epidemic.

Hybrid vaccination, also known as heterologous vaccination, has been described in the art. For example, Cohn BA, Cirillo PM, Murphy CC, Krigbaum NY, Wallace AW: SARS-CoV-2 vaccine protection and deaths among US veterans during 2021. Science 2022, 375(6578):331-336 describes a study of hybrid vaccination comprising a systemic subcutaneous prime followed by oral boosts with the LTB subunit to enhance the immunogenicity of a vaccine. In the study, SARS-CoV-2 nucleocapsid serving as a T-cell antigen is covalently linked to LTB. The nucleocapsid is required for activating Th-1 and eliciting IL-2 expression.

Another study (Lucas C, Vogels CBF, Yildirim I, Rothman JE, Lu P, Monteiro V, Gehlhausen JR, Campbell M, Silva J, Tabachnikova A, et al: Impact of circulating SARS-CoV-2 variants on mRNA vaccine-induced immunity. Nature 2021, 600(7889):523-529) revealed elevated protective immunity by systemic prime-mucosal boost immunization. In this study, animals were primed by the mRNA vaccine and boosted with the adenovector vaccine through mucosal routes. The adenovirus vector is a replication-deficient virus with target antigen(s) inserted. After administration, the adenovector infects the cell and dumps its viral DNA genome into the nucleus for transcription. Invasion of adenovector to host cells also induces innate immunity-mediated inflammation, and the burst of pro-inflammatory IL-1, IL-6, TNF-α, etc. drives the anti-insert immunity. Also, complexity promotes immunogenicity. In addition to the insert being targeted by immunity, the entire adenovirus virion complex is targeted by vaccination.

Vaccines play an important role in the prevention of infectious diseases. In view of the ongoing pandemic of mucosal viruses such as SARS-CoV-2 and seasonal influenza, there remains a need for an effective and patient compliant (such as non-invasive) booster for regular vaccination.

### Summary of the Invention

Parenteral vaccines mediate strong systemic immunity against SARS-CoV-2 but show minimal effect on secretory IgA (sIgA), tissue-resident memory B (BRM), and memory T (TRM) cell activation. Three years after the onset of the pandemic, over 71% of the global population has received at least one dose of the vaccines for SARS-CoV-2 infection via parental routes. Nevertheless, transmission of the disease is still ongoing due to high mutation rates of the virus and inadequate protection to upper respiratory epithelial cells by current vaccination. Thus, illness of the upper respiratory tract has not been prevented effectively. Because mucosal immunity is one potential solution to the current pandemic, we developed an intranasal booster that effectively induces serum IgG, serum IgA, and mucosal IgA against the virus. The booster can be applied to a subject treated with an intranasal prime or an intramuscular prime, the latter referred to as a hybrid vaccination regimen. Both the intranasal-prime-intranasal-boost and the hybrid vaccination regimen, which involves intramuscular prime and intranasal boost, enhance serum-neutralizing activity and elicit robust mucosal neutralizing activity. The LTh(αK)-adjuvanted SARS-CoV-2 spike protein intranasal (IN) booster is found to significantly augment neutralizing antibody titers in serum and nasal wash samples. This LTh(αK)-adjuvanted IN booster enhances Th1/Th2 balanced humoral and mucosal immunity in a mouse model. The IN booster, which enhances mucosal and circulatory immunogenicity, brings clinically meaningful mucosal defense for vaccination.

In addition to inducing immunity against SARS-CoV-2, the IN booster of the present invention is also effective in inducing both humoral and mucosal immunities against influenza viruses using influenza antigen adjuvanted with LTh(αK). LTh(αK) is found to be an effective adjuvant in eliciting systemic and mucosal immunities against mucosal viruses in a booster. Compared to conventional IM boosters, the LTh(αK) IN booster is more effective in preventing mucosal virus infections and blocking the transmissions of viruses.

The present invention is described in detail in the following sections. Other characterizations, purposes and advantages of the present invention can be easily found in the detailed descriptions and claims of the invention.

### Brief Description of the Drawings

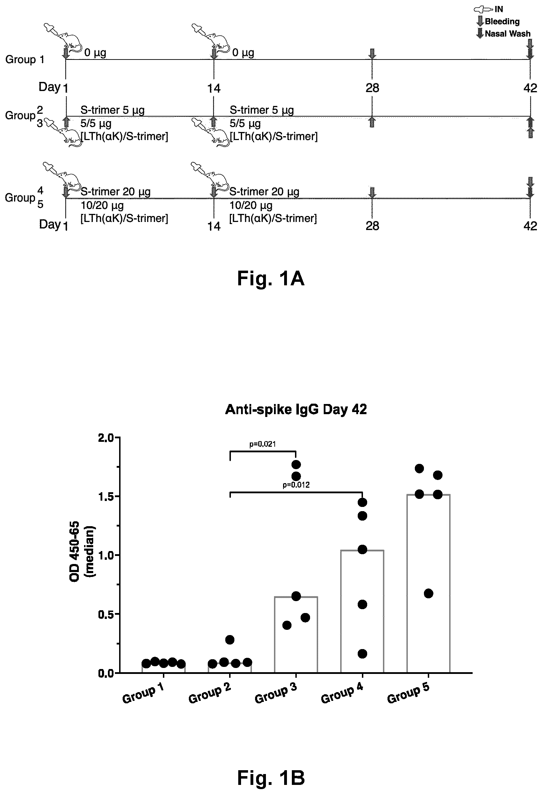

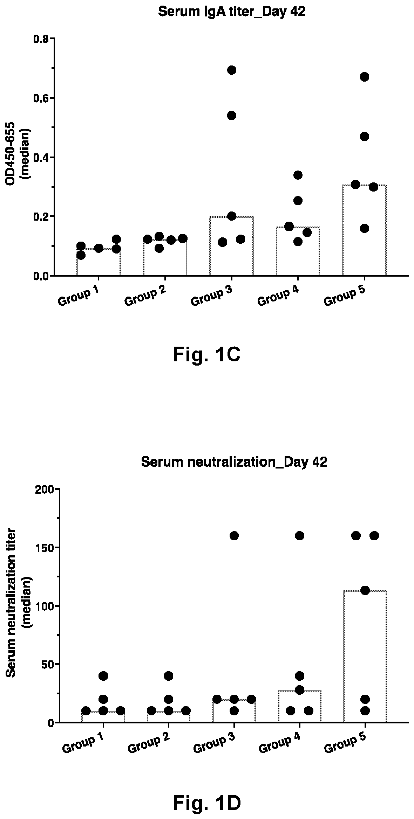

Fig. 1 shows the immunization scheme and immune responses to SARS-CoV-2 spike antigen. A) Intranasal immunization with unadjuvanted or LTh(αK) adjuvanted S-trimer. B-C) Immunization-induced S-specific serum IgG and IgA. Sera were 90- and 30-fold diluted for the analysis of S-specific IgG and IgA titers, respectively. D) Serum neutralization assay showed enhanced neutralizing activity for S-trimer adjuvanted with LTh(αK). Kruskal-Wallis test results are highlighted in bold lines.

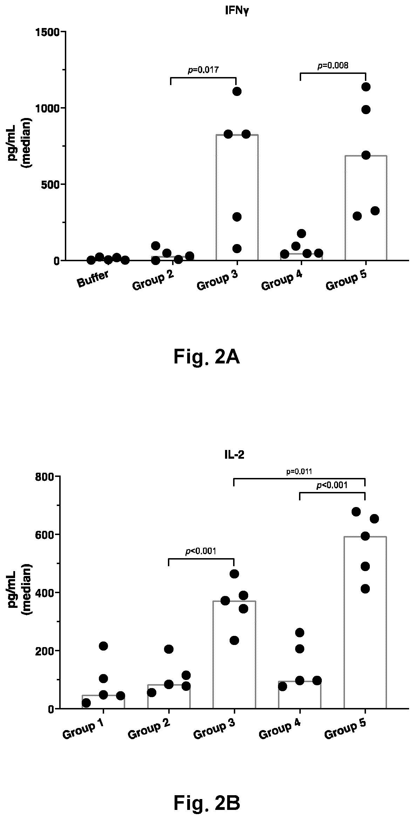

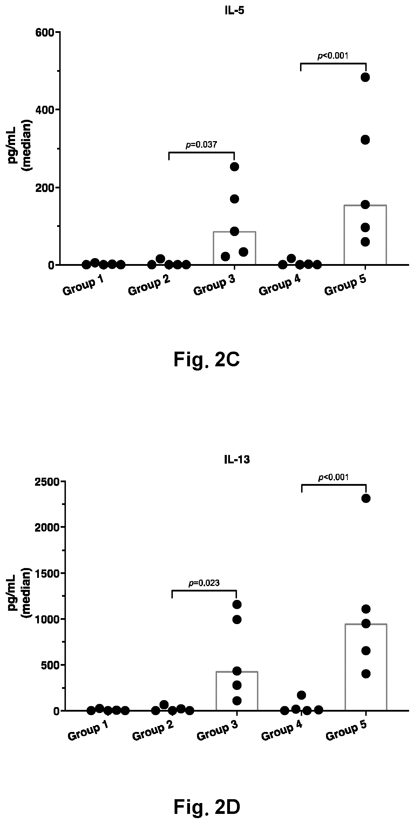

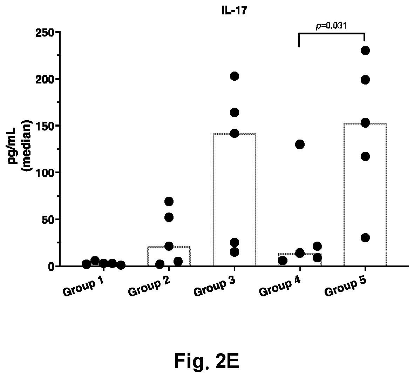

Figs. 2A-2E show the increased production of Th1, Th2 and Th17 cytokines by intranasal immunization with LTh(αK)-adjuvanted S-trimer vaccines.

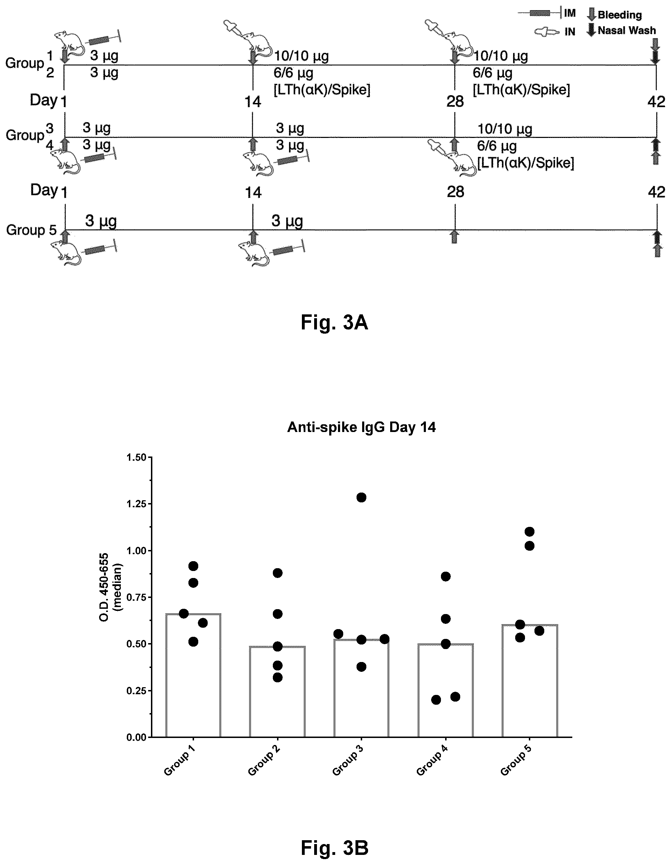

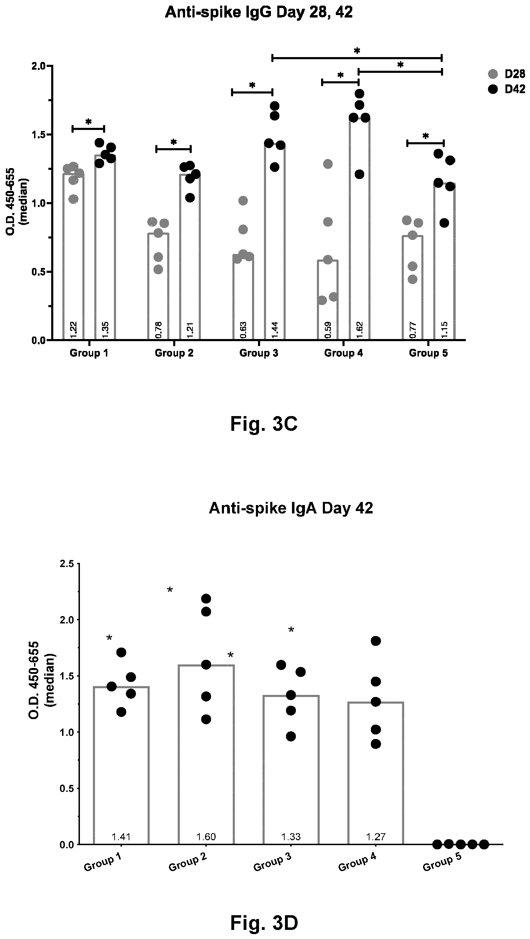

Fig. 3 shows the immunization scheme and immune responses to MVC-COV-1901. A) Combinations of intramuscular prime and intranasal boost, intramuscular prime and intramuscular boost, and intranasal prime and intranasal boost immunizations. B) S-2P specific serum IgG on Day 14. Sera were 4,000-fold diluted. C) S-2P specific serum IgG on Days 28 and 42. Sera were 16,000-fold diluted. D) S-2P specific IgA titer on Day 42 from nasal wash. Nasal washes were 10 fold-diluted. Kruskal-Wallis test results are highlighted in bold lines.

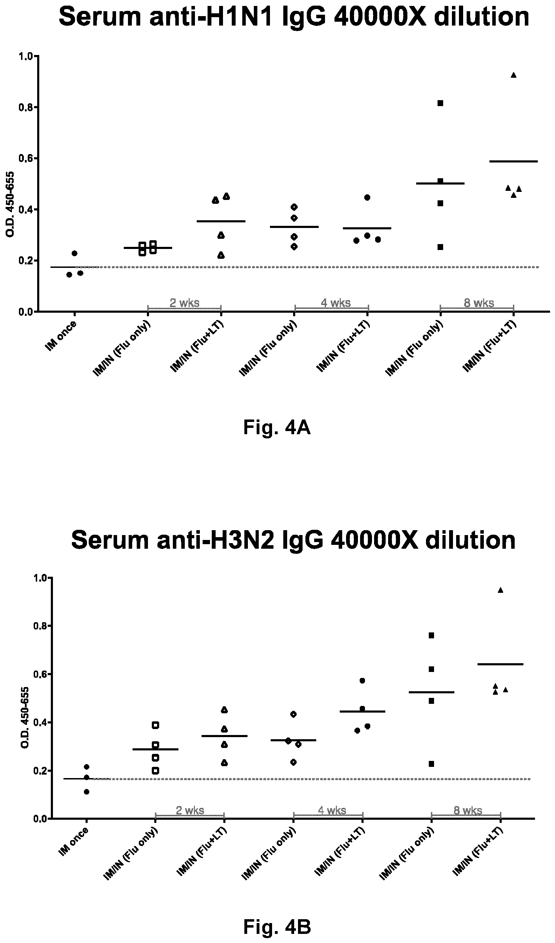

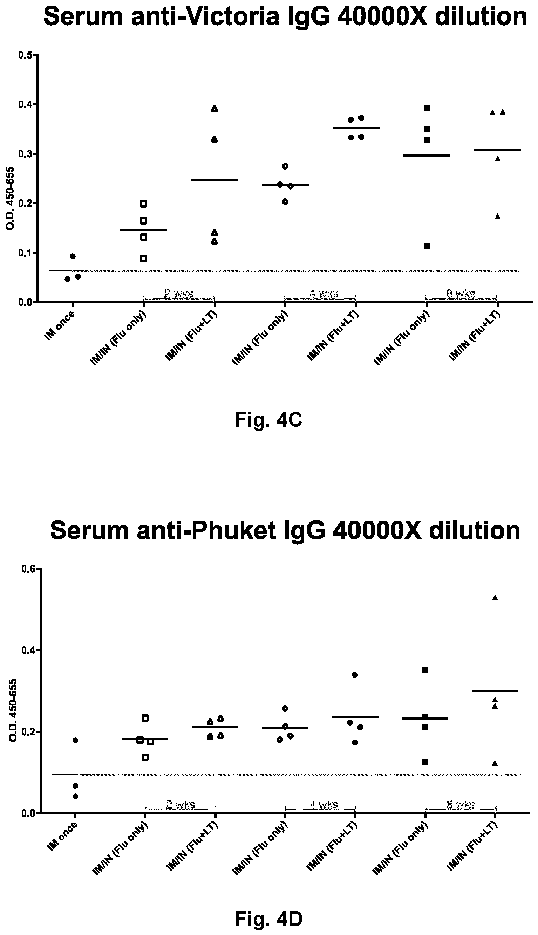

Figs. 4A-4D show the levels of serum IgG against the seasonal influenza viruses H1N1, H3N2, B-Victoria and B-Phuket antigens from hybrid vaccination.

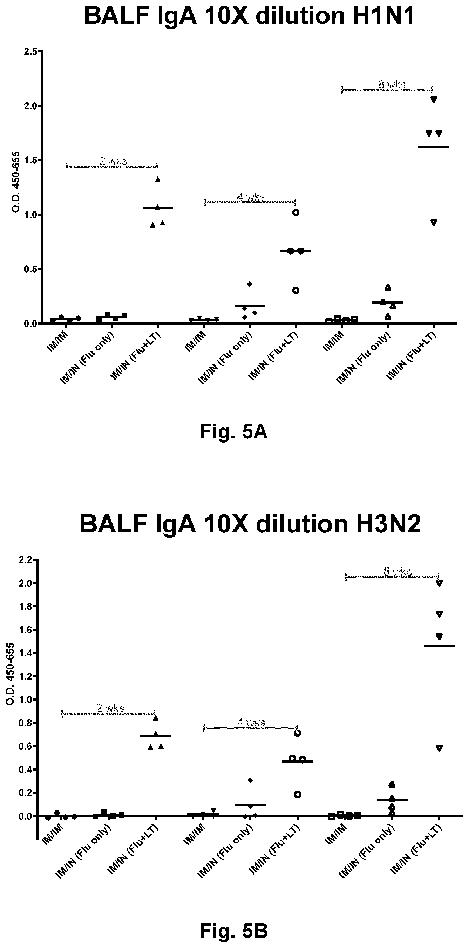

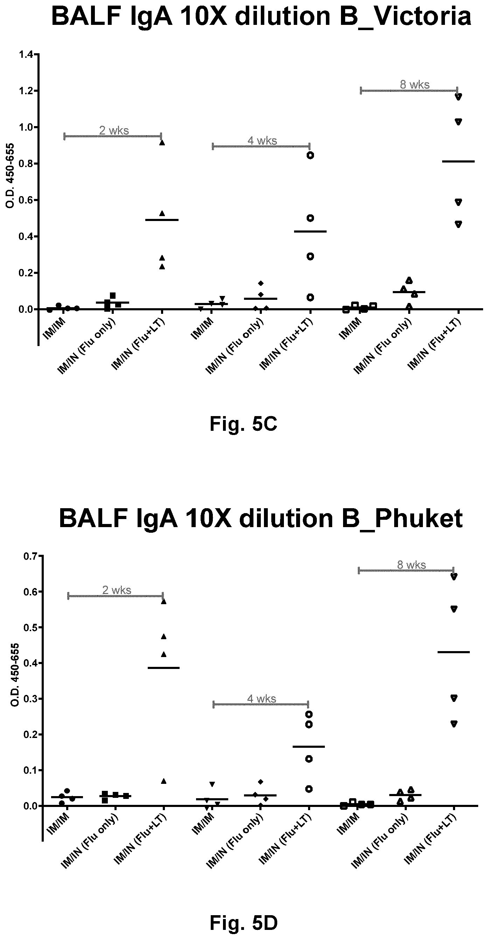

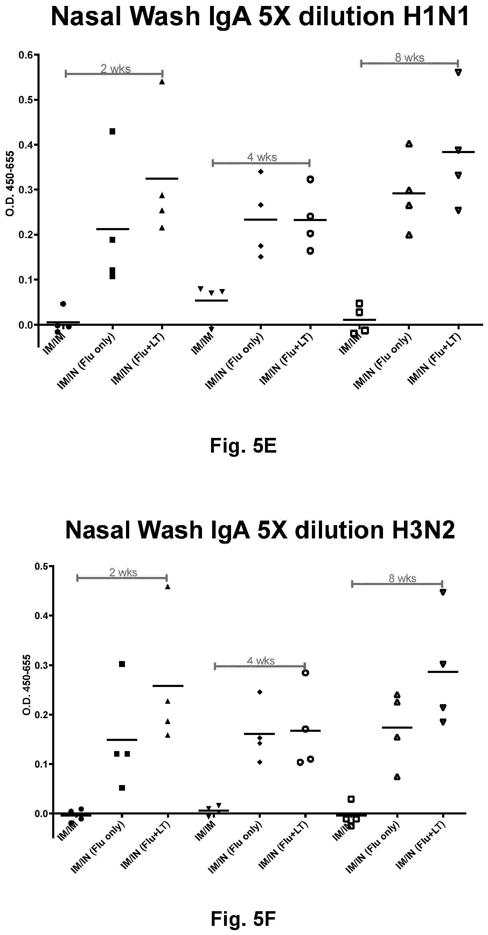

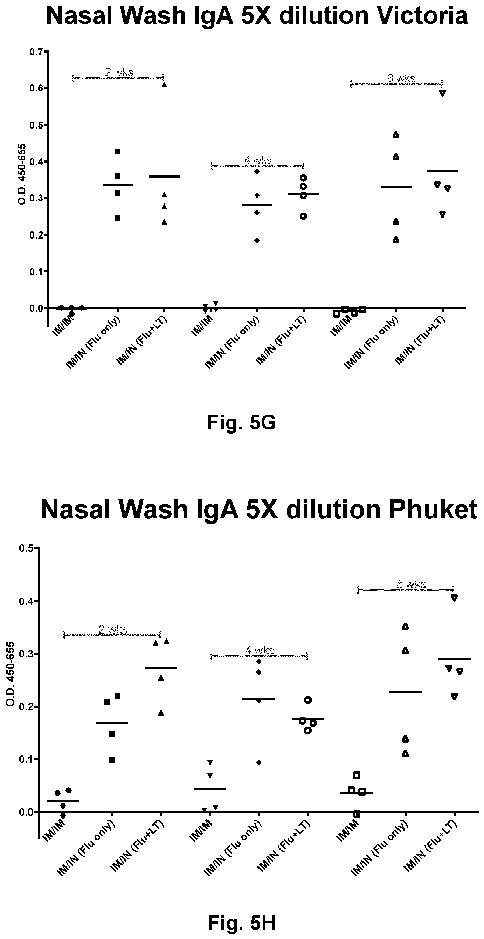

Figs. 5A-5H show the levels of secretory IgA antibody against the seasonal influenza viruses H1N1, H3N2, B-Victoria and B-Phuket antigens from hybrid vaccination by BALF specimen.

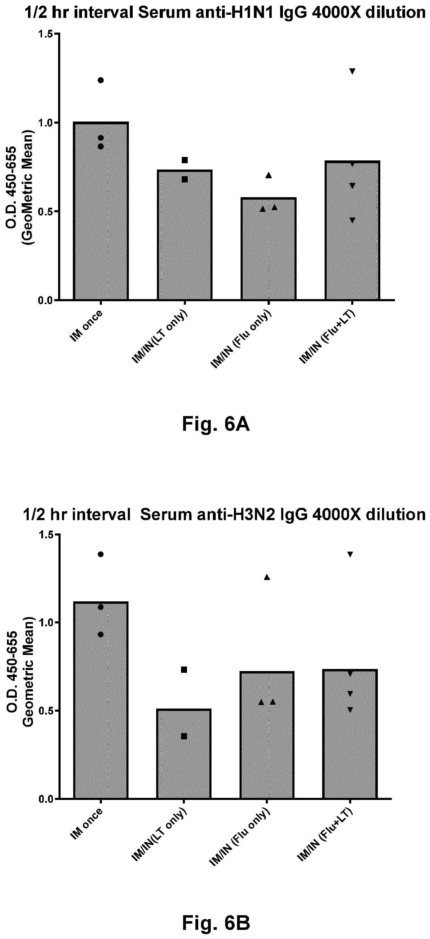

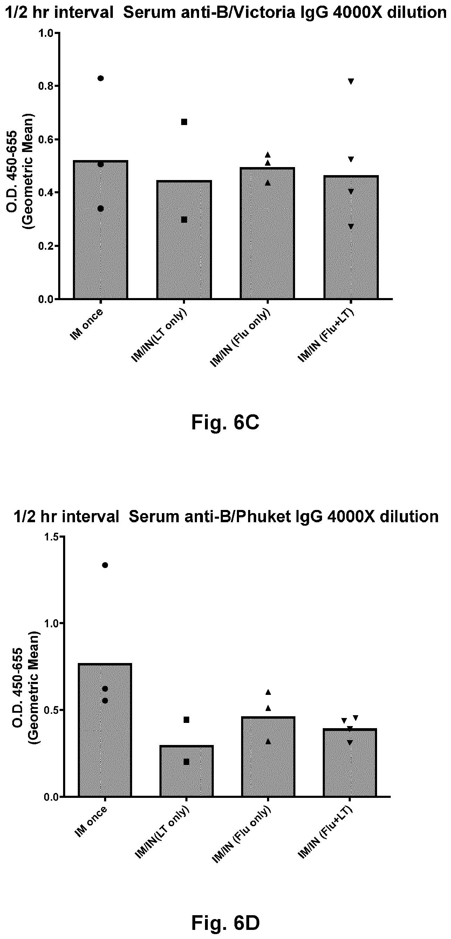

Figs. 6A-6D show the results of short interval (1/2 hour) between IM prime and IN boost on the immunogenicity against influenza antigens. "IM" refers to intramuscular vaccination. All prime doses were administered through IM. "IM/IN (LT only)" refers to intramuscular prime and intranasal boost with LTh(αK) without flu antigens. "IM/IN (Flu+LT)" refers to intramuscular prime and intranasal boost with LTh(αK) with flu antigens.

### Detailed Description of the Invention

Unless otherwise defined herein, scientific and technical terms used in connection with the present invention shall have the meaning commonly understood by those of ordinary skill in the art. The meaning and scope of the terms should be clear; however, in the event of any latent ambiguity, definitions provided herein take precedence over any dictionary or extrinsic definition.

As utilized in accordance with the present disclosure, the following terms, unless otherwise indicated, shall be understood to have the following meanings.

Throughout this specification, the word "comprise" or variations such as "comprises" or "comprising" will be understood to imply the inclusion of a stated integer (or components) or group of integers (or components), but not the exclusion of any other integer (or components) or group of integers (or components).

The singular forms "a," "an," and "the" include the plurals unless the context clearly dictates otherwise. Unless otherwise required by context, plural terms shall also include the singular.

"Treatment," "treating," and the like refer to an approach for obtaining a beneficial or desired result, including clinical results. For purposes of this disclosure, beneficial or desired results include but are not limited to inhibiting and/or suppressing the onset and/or development of a condition or reducing the severity of such condition, such as reducing the number and/or severity of symptoms associated with the condition, increasing the quality of life of those suffering from the condition, decreasing the dose of other medications required to treat the condition, enhancing the effect of another medication a patient is taking for the condition, and/or prolonging survival of patients having the condition.

"Prophylaxis," "prophylactic," "prevent," "preventing," "prevention," and the like refer to reducing the probability of developing a condition in a patient who does not have but is at risk of developing a condition. A patient "at risk" may or may not have a detectable condition, and may or may not have displayed a detectable condition prior to the treatment methods disclosed herein.

"Administering" or "administration of" a substance, a compound or an agent to a subject can be carried out using one of a variety of methods known to those skilled in the art. For example, a compound or an agent can be administered intranasally. Administering can also be performed, for example, once, a plurality of times, and/or over one or more extended periods. In some aspects, the administration includes both direct administration, including self-administration, and indirect administration, including the act of prescribing a drug. For example, as used herein, a physician who instructs a patient to self-administer a drug, or to have the drug administered by another and/or who provides a patient with a prescription for a drug is administering the drug to the patient.

The term "modulator" used herein refers to the agent that regulates a condition, level, or amount. The regulation may be upregulation or downregulation.

The term "humoral immune response" used herein refers to immune responses that are induced at the serum. For example, humoral immune response includes, but is not limited to, antigen-specific immunoglobulin G and its subclasses, immunoglobulin A and its subclasses, immunoglobulin M and its subclasses, and cell-mediated immunity to immunized antigens.

The term "mucosal immune response" used herein refers to immune responses that are induced at the mucosa. For example, mucosal immune response includes, but is not limited to, antigen-specific immunoglobulin G and its subclasses, immunoglobulin A and its subclasses, immunoglobulin M and its subclasses, and cell-mediated immunity to immunized antigens.

The terms "patient," "subject," or "individual" are used interchangeably and refer to either a human or a non-human animal. These terms include mammals, such as humans, primates, livestock animals (including bovines, porcines, etc.), companion animals (e.g., canines, felines, etc.), and rodents (e.g., mice and rats).

"Effective amount" refers to such amount of a therapeutic agent or a pharmaceutically acceptable salt thereof which, in consideration of its parameters of efficacy and potential for toxicity, as well as based on the knowledge of the practicing specialist, should be effective in a given therapeutic form. As is understood in the art, an effective amount can be administered in one or more doses.

"Hybrid vaccination" refers to a vaccination scheme wherein the booster is administered through a different route from the prime vaccine. For example, a hybrid vaccination comprises an intramuscularly administered prime vaccine and an intranasally administered booster vaccine.

"Mucosal virus" refers to a virus that infects a subject through the mucosa. For example, a mucosal virus may infect through the mucosa of the eyes, nose, mouth, respiratory tract, and other anatomical mucosa. Exemplary mucosal viruses are coronaviruses, influenza viruses, rhinoviruses (HRV), adenoviruses, respiratory syncytial viruses, enteroviruses, parainfluenza viruses, paramyxoviruses, human metapneumoviruses, human bocaviruses, etc.

In the present invention, it was surprisingly found that an intranasal booster comprising a detoxified *Escherichia coli* labile toxin (LT), such as LTh(αK), and an antigen from a mucosal virus, such as coronavirus and influenza virus, can elicit both strong humoral and mucosal immune responses. Therefore, the present invention relates to an intranasal booster comprising a detoxified *Escherichia coli* labile toxins (LT) and an antigen from a mucosal virus.

The present disclosure relates to a method for enhancing humoral and mucosal immunities and Th1, Th2 and Th17 cytokine productions, leading to Th1/Th2 balance. Therefore, the present disclosure provides a method of vaccinating a subject using an intranasal booster comprising a detoxified *Escherichia coli* labile toxin (LT) and an antigen from a mucosal virus.

In one embodiment, the detoxified LT is LTh(αK). LTh(αK) corresponds to LTS61K as disclosed in US 2008/0102078, which is a detoxified *E. coli* LT holotoxin with a lysine substitution at the position corresponding to position 61 of SEQ ID NO: 5 as disclosed in US 2008/0102078.

In one embodiment, the coronavirus is a virus of the family of *Orthocoronavirinae.* In a preferred embodiment, the coronavirus is SARS-CoV, MERS-CoV, or SARS-CoV-2. In a more preferred embodiment, the coronavirus is SARS-CoV-2.

In one embodiment, the antigen from coronavirus is a spike protein. In a preferred embodiment, the spike may be an immunogenic portion of the spike. In a more preferred embodiment, the immunogenic portion of the spike may be recombinant. In a still preferred embodiment, the recombinant antigen may be a multi-mer such as a tri-mer.

In one embodiment, the influenza virus is a virus of the family of *Orthomyxoviridae.* In a preferred embodiment, the influenza virus is seasonal influenza virus. In a more preferred embodiment, the influenza virus is A/Victoria/2570/2019 (H1N1)pdm09-like virus; A/Darwin/9/2021 (H3N2)-like virus; B/Austria/1359417/2021 (B/Victoria lineage)-like virus; or B/Phuket/3073/2013 (B/Yamagata lineage)-like virus.

In one embodiment, the detoxified *Escherichia coli* labile toxin (LT) is administered to a subject who has been previously primed. In another embodiment, the subject has been primed with coronavirus challenge. In another embodiment, the subject has been primed with influenza virus challenge. In one preferred embodiment, the subject has been primed through intramuscular route. In one preferred embodiment, the subject has been primed through intranasal route.

In one embodiment, the intranasal booster is administered to the subject one or more times after the subject has been primed with coronavirus or influenza virus challenge. In a preferred embodiment, the intranasal booster is administered to the subject one time after the subject has been primed with coronavirus or influenza virus challenge. In another preferred embodiment, the intranasal booster is administered to the subject two times after the subject has been primed with coronavirus or influenza virus challenge. In another preferred embodiment, the intranasal booster is administered to the subject multiple times after the subject has been primed with coronavirus or influenza virus challenge. In another preferred embodiment, the intranasal booster is administered to the subject regularly or periodically after the subject has been primed with coronavirus or influenza virus challenge.

In one embodiment, the intranasal booster elicits both humoral and mucosal immune responses through enhancing one or more of serum IgG, serum IgA and mucosal IgA levels. In one preferred embodiment, the enhanced humoral and mucosal immune responses increase neutralizing antibody titers.

In one embodiment, the intranasal booster enhances Th1, Th2 and Th17 cytokine production by increasing the levels of IFN-y, IL-2, IL-5, IL-13, and IL-17.

In one embodiment, the increased humoral and mucosal immune responses involve upregulation of immune components. In another embodiment, the increased humoral and mucosal immune responses involve downregulation of immune components. More preferably, the increased humoral and mucosal immune responses provide a prophylactic or preventive benefit.

The present disclosure further provides a method for vaccinating a subject against a mucosal virus infection, comprising administering to the subject an immunologically effective amount of an intranasal booster as described herein, wherein the subject has been previously primed. In one embodiment, the method of the disclosure provides the efficacies as described herein.

The present disclosure provides an intranasal booster that is non-invasive and convenient to use for a subject who has been intramuscularly or intranasally primed by a coronavirus or influenza virus vaccine, or a subject who has been so primed and received one or more intramuscular or intranasal booster vaccines. Since the subject can administer the intranasal booster on its own, the burden to the medical care system to vaccinate the population can be greatly reduced. Moreover, since the intranasal booster is non-invasive, the subject would be more willing or compliant to take the vaccination. The present invention provides a novel intranasal booster comprising simple ingredients (LTh(αK) and the antigen) for the prophylaxis of coronavirus or influenza virus infection and a method of vaccinating a subject using the intranasal booster. The present invention facilitates the development of novel booster vaccine for coronavirus or influenza virus infection beyond traditional intramuscular means.

Having now generally described the invention, the same may be more readily understood through reference to the following examples, which provide exemplary protocol for performing the method of the present invention in the prophylaxis of coronavirus or influenza virus infection. The examples are offered for illustrative purposes only, and are not intended to limit the scope of the present invention in any way. Efforts have been made to ensure accuracy with respect to numbers used (e.g., amounts, temperatures, etc.), but some experimental error and deviation should, of course, be allowed for.

### Examples

### Materials and Assays

### Production of S-2P protein ectodomains

The production of SARS-CoV-2 spike trimer for the S-prefusion form of the Wuhan strain was prepared as previously described by (Liao HC, Wu WL, Chiang CY, Huang MS, Shen KY, Huang YL, Wu SC, Liao CL, Chen HW, Liu SJ: Low-Dose SARS-CoV-2 S-Trimer with an Emulsion Adjuvant Induced Th1-Biased Protective Immunity. Int J Mol Sci 2022, 23(9)). Briefly, SARS-CoV-2 S proteins with a prefusion form were designed to contain a nonfunctional furin cleavage site and two stabilizing prolines in the hinge loop. The transmembrane domain and the C-terminal intracellular tail were replaced by a trimerization domain IZN4 (S-Trimer), followed by histidine (8-mer) at the carboxy terminus for purification. Both S variant DNA constructs were cloned into the pcDNA3.1(+) plasmid vector by GenScript (Piscataway, NJ, USA). The production of the S-Trimer was compiled using the ExpiCHO^{™} Expression System Kit (ThermoFisher Scientific, Carlsbad, CA, USA). Briefly, the S-Trimer (or S-2P) was transiently expressed by ExpiCHO cells according to the manufacturer's instructions. The S-Trimer-containing medium was centrifuged and dialyzed before purification through a Ni2+-NTA agarose column (GE) with 0.5 M imidazole. This antigen was used in Examples 1 and 2.

The SARS-CoV-2 spike trimer for the MVC-COV1901 vaccine was prepared as previously described (Kuo TY, Lin MY, Coffman RL, Campbell JD, Traquina P, Lin YJ, Liu LT, Cheng J, Wu YC, Wu CC et al: Development of CpG-adjuvanted stable prefusion SARS-CoV-2 spike antigen as a subunit vaccine against COVID-19. Sci Rep 2020, 10(1):20085). Briefly, the plasmid expressing recombinant S-2P ectodomain of SARS-CoV-2 was transfected to ExpiCHO-S cells (Thermo Fisher Scientific). The secreted S-2P protein was purified by affinity chromatography and tags were removed by HRV3C protease followed by second S-2P purification. The purified S-2P proteins were quantified by BCA assay (Thermo Fisher Scientific), flash-frozen in liquid nitrogen, and then stored at -80 °C before use. This antigen was used in Examples 3 and 4.

### Mucosal Sample collections

Nasal washes were obtained 2 weeks post boosting on Day 42. Nasal washes were collected from decapitated animals by back-flushing 1 ml PBS from the trachea. Collected nasal washes were centrifuged and the nasal fluids were stored at -20°C before assays.

### Measurement of SARS-Co V-2 recombinant S-specific antibodies

The anti-SARS-CoV-2 S-specific serum IgG and nasal sIgA antibodies were determined by enzyme-linked immunosorbent assay (ELISA). The 96-well high binding plates (Thermo 442404) were coated with 100 µL of 5µg/mL recombinant trimeric S protein diluted in carbonate coating buffer (pH 9.5) overnight at 4°C. The plates were washed three times using phosphate buffer saline (PBS) containing 0.05% Tween 20 (PBST) and the wells were blocked with 5% skim milk at 37°C for 1 h. Serially diluted serum was added to the plate, 100 µL/well, followed by a 1-hour incubation at 37°C. Following three washes, 100 µL of anti-mouse horseradish peroxidase-conjugated anti-IgG (Jackson ImmunoResearch, 115-035-062, 5000X dilution) or IgA antibody (KPL 5220-0367, 2500X dilution) dilution at 5% skim milk was added with a 1-hour incubation followed by three rinses with PBST. At the end of the wash, 100 µL of 3,3',5,5'-tetramethyl biphenyl diamine (TMB) was added to the wells and the color reaction lasted for 5 minutes. The reaction was quenched with a stopping solution and optical density was measured at OD 450-655.

### Pseudovirus production and titration

Pseudovirus expressing full-length wild-type SARS-CoV-2 S protein was prepared as described in (Kuo TY, Lin MY, Coffman RL, Campbell JD, TraquinaP, Lin YJ, Liu LT, Cheng J, Wu YC, Wu CC et al: Development of CpG-adjuvanted stable prefusion SARS-CoV-2 spike antigen as a subunit vaccine against COVID-19. Sci Rep 2020, 10(1):20085). Briefly, full-length wild-type Wuhan-Hu-1 strain SARS-CoV-2 spike protein-expressing plasmid was transfected into HEK293T cells with packaging and reporter plasmids pCMVΔ8.91 and pLAS2w.FLuc.Ppuro (RNAi Core, Academia Sinica, Taiwan) using TransIT-LT1 transfection reagent (Mirus Bio). Mock pseudoviruses were produced by omitting the p2019-nCoV spike (WT). At 72 hours post-transfection, supernatants were collected, filtered, and stored at -80°C. The transduction unit (TU) of SARS-CoV-2 pseudotyped lentivirus was calculated by viability assay in response to the limited dilution of lentivirus.

### Pseudovirus-based neutralization assay

Pseudovirus-based neutralization was prepared as described in Kuo TY, Lin MY, Coffman RL, Campbell JD, Traquina P, Lin YJ, Liu LT, Cheng J, Wu YC, Wu CC et al: Development of CpG-adjuvanted stable prefusion SARS-CoV-2 spike antigen as a subunit vaccine against COVID-19. Sci Rep 2020, 10(1):20085. Briefly, HEK293-hAce2 cells (2×10⁴ cells/well) were seeded in 96-well plate followed by additional heat-inactivated, MEM-diluted mouse sera or nasal washes. The initial dilution factor to serum samples was 20, and then two-fold serial dilutions were carried out. Due to high dilution for nasal wash specimens, nasal wash neutralization was initiated at two-fold serial dilution. The diluted specimens were mixed with an equal volume of pseudovirus (1000 TU) and incubated before being added to the plates with cells. After incubation, the culture medium was replaced with a fresh medium. On the next day, the replacement of the culture medium was repeated, cells were lysed at 72 h post infections, and relative luciferase units (RLU) were measured. The luciferase activity was detected by Tecan i-control (Infinite 500). The 50% and 90% inhibition dilution titers (ID50 and ID90) were calculated.

### Cytokine analysis

Cytokine analysis was prepared as described in Liao HC, Wu WL, Chiang CY, Huang MS, Shen KY, Huang YL, Wu SC, Liao CL, Chen HW, Liu SJ: Low-Dose SARS-CoV-2 S-Trimer with an Emulsion Adjuvant Induced Th1-Biased Protective Immunity. Int J Mol Sci 2022, 23(9). Briefly, splenocyte cytokine production was assessed using cytokine ELISA. A spleen was isolated from individual mice in each group four weeks after the second IN vaccination. Splenocytes were prepared by centrifugation of mesh-homogenized spleens in RPMI-1640 medium and suspended in LCM with 10% fetal bovine serum. The obtained splenocytes were cultured with recombinant SARS-CoV-2 S (10 µg/mL) in 24-well plates (5 × 10⁶ cells/well) at 37°C under 5% CO₂. After 4 days, the supernatant was collected to quantify the production levels of Th1 cytokines (IFN-y and IL-2) and Th2 cytokines (IL-5 and IL-13), and Th17 cytokine (IL-17A) by using different cytokine ELISA kits (ThermoFisher Scientific, Carlsbad, CA, USA) as described by the manufacturer's instructions.

### Statistical analysis

GraphPad Prism 9 was used to perform statistical analysis. A Kruskal-Wallis test (bold-face) with Dunn's multiple comparisons test was used to assess statistical differences among groups. A two-tailed unpaired Student's t-test was used to evaluate the differences between treatments.

### Example 1: Intranasal (IN) immunization of mice using LTh(αK)-adjuvanted SARS-CoV-2 spike significantly enhances spike-specific (S-specific) serum IgG and IgA levels

Intranasal vaccination of LTh(αK)-adjuvanted S-trimer vaccine elevates both SARS-CoV-2 spike-specific (S-specific) IgG and IgA. The study procedure is outlined in Fig. 1A. In the vaccination scheme described in Example 1 (Fig. 1A), female mice, 4- to 6-week old Balb/c, were purchased from BioLASCO Taiwan and randomly divided into five groups, 5 mice in each group. All mice received two IN administrations (nasal instillation), primed on Day 1 and boosted on Day 14. Group 1 to Group 5 received saline, 5 µg S-trimer, 5 µg S-trimer+5 µg LTh(αK), 20 µg S-trimer, 20 µg S-trimer+10 µg LTh(αK), respectively. Mice were bled on Days 1, 14, and 28, and sacrificed on Day 42. The splenocytes from all animals were collected on the day of sacrifice. Serum IgG levels on Day 42 were analyzed by ELISA. The results showed that the level of S-specific serum IgG increased as the dose of S-trimer increased, as shown in Group 2 vs. Group 4, and Group 3 vs. Group 5 in Fig. 1B. In addition, adjuvanting the spike with LTh(αK) in the IN vaccines further enhanced anti-S IgG titers, as shown in Group 2 vs. Group 3 and Group 4 vs. Group 5. A similar effect was also found for serum S-specific IgA (Fig. 1C). The enhanced S-specific IgA titers can be seen in Groups 3, 4, and 5, wherein Groups 3 and 5 in which the IN vaccines adjuvanted with LTh(αK) demonstrated the highest enhancement. On the other hand, Groups 3 and 5 showed that the amount of the spike antigen did not significantly influence S-specific IgA titers when adjuvanted with LTh(αK). This confirms the effectiveness of LTh(αK) as a mucosal adjuvant. Moreover, the serum neutralizing analysis (Fig. 1 D) showed that LTh(αK) enhanced serum S-specific neutralization.

### Example 2: Intranasal boosting with LTh(αK) adjuvanted SARS-CoV-2 S-trimer activated Th1, Th2 and Th17

Balanced Th1 and Th2 cytokine profiles were revealed after intranasal instillation of the LTh(αK) adjuvanted vaccine. Following the immunization scheme of Fig. 1A, on study Day 42, mice were sacrificed and the splenocytes were harvested and incubated with S-trimer. After 4 days of incubation, the levels of IFN-γ, IL-2, IL-5, IL-13, and IL-17 were analyzed by ELISA. The levels of Th1, Th2 and Th17 cytokines were found to be enhanced (Figs. 2A-E) after administration with LTh(αK) adjuvanted intranasal vaccines following the priming and boosting scheme shown in Fig. 1A. After IN administration of LTh(αK) adjuvanted vaccine, the levels of IFN-y, IL-2, IL-5, IL-13, and IL-17 were elevated significantly (Fig. 2A-E). All cytokines were significantly higher in animals receiving LTh(αK) but not in the S-trimer-only group, confirming that the LTh(αK) is a potent IN immune modulator to Th1, Th2, and Th17. Increasing the amount of the S-trimer antigen did not result in significant enhancement of the cytokines. The level of IL-2 was enhanced significantly by increasing the amount of LTh(αK) (Fig. 2B). The levels of IL-5, IL-13, and IL-17 in Group 5 also showed numerical gain by increased amount of LTh(αK) (Figs.2C-E). Administration of recombinant S-trimer alone, either at high dose or low dose, showed minimal influence on the expression of Th1, Th2 and Th17 cytokines.

### Example 3: Intranasal boosting with LTh(αK)-adjuvanted SARS-CoV-2 spike for intramuscular primed mice enhances circulating IgG and secretory IgA

LTh(αK)-adjuvanted IN booster enhances spike-specific sIgA and serum IgG. To explore the mucosal immunity enhancement without undermining serum IgG, we primed mice with MVC-COV1901, a SARS-CoV-2 S-2P subunit vaccine adjuvanted with aluminum hydroxide and CpG1018, followed by intranasal boosting with LTh(αK) adjuvanted S-2P. In the vaccination scheme described in Example 3 (Fig. 3A), female mice, 6- to 8-week old Balb/c, were purchased from BioLASCO Taiwan and randomly divided into five groups, 6 mice in each group, and received immunization via IM only, or combination routes of IM and IN as illustrated in Fig. 3A. On study Day 1, animals in all groups were primed intramuscularly with MVC-COV-1901 containing 3 µg S-2P (Lin YJ, Lin MY, Chuang YS, Liu LT, Kuo TY, Chen C, Ganesan S, Fattom A, Bitko V, Lien CE: Protection of hamsters challenged with SARS-CoV-2 after two doses of MVC-COV1901 vaccine followed by a single intranasal booster with nanoemulsion adjuvanted S-2P vaccine. *Sci Rep* 2022, 12(1):11369). On study Day 14, animals in Groups 1 and 2 were boosted intranasally with LTh(αK) adjuvanted S-2P, and Groups 3-5 were boosted intramuscularly with MVC-COV-1901 containing 3 µg S-2P. MVC-COV-1901 is a vaccine approved under EUA by Taiwan Food and Drug Administration (TFDA) containing S-2P as antigen and adjuvanted with CpG1018 plus aluminum hydroxide (S-2P/CpG1018/Aluminum hydroxide).

As shown in the scheme in Fig. 3A, mice were randomly divided and received vaccination via IM (prime) and IM (boost) only, or a hybrid vaccination of IM (prime) and IN (boost). IM priming elevated S-specific IgG on Day 14 in all groups (Fig. 3B). The IN and the IM booster were each highly efficacious in enhancing S-specific IgG titers in IM primed mice (Fig. 3C). On Day 28, sera from all animals were collected prior to addition of boosters. The ELISA analysis of Day 28 sera showed that anti-S IgG titers induced by LTh(αK)-adjuvanted IN booster were equivalent (Group 2, Fig. 3C) or better (Group 1, Fig. 3C) compared to IM booster (Groups 3-5, Fig. 3C). Additional LTh(αK) adjuvanted booster on Day 28 significantly enhanced the S-specific IgG titers in Groups 1, 3 and 4 on Day 42 (Fig. 3C). Following additional IN boosting on Day 28, mice developed robust S-specific sIgA titers regardless of prior IM or IN boost (Groups 1-4, Fig. 3D). The IgA titers to S-2P were not affected by the number of IN doses or the amount of S-2P in the booster. The S-specific sIgA was within background level for IM-only mice (Group 5, Fig. 3D).

### Example 4: Intranasal boosting with LTh(αK)-adjuvanted SARS-CoV-2 spike induces high levels of neutralizing antibodies in serum samples and nasal wash

LTh(αK)-adjuvanted IN booster is potent in mediating the production of S-specific neutralizing antibodies in serum and nasal mucosa. Inhibition of pseudoviruses carrying wild-type spike protein by mice sera primed and boosted by IM, IN, or IM and IN was tested. All mice sera and nasal washes were collected on Day 42 and analyzed for pseudovirus-blocking activity. Neutralizing titers by pseudovirus assay were determined based on both sera (TCID₉₀) and nasal washes (TCID₅₀). The results are shown in Table 1 below. On Day 14, two weeks post-priming, serum-neutralizing titers were not elevated in most mice except for some minimal elevation in Group 1. On day 28, two weeks post-first boosting, significantly elevated serum neutralizing titers were revealed among different groups. The neutralizing activities of pseudovirus via IN boosting (Groups 1 and 2) are not different from IM-boosted groups (Groups 3-5). The serum-neutralizing antibody titers in Groups 1-4 on Day 42 are much higher than those on Day 28. The results show that IN booster is as efficacious as IM booster, and a second IN booster effectively further increases serum-neutralizing antibody titers.

**Table 1: serum neutralizing results**

| | Administration Route | | | Pseudovirus Neutralizing Assay ID90/GMT | | |
|---|---|---|---|---|---|---|
| Groups | 1st | 2nd | 3rd | 1st dose | 2nd dose | 3rd dose |
| 1 | IM | IN | IN | 27.0 | 581.2 | 5550.9 |
| 2 | IM | IN | IN | 20.0 | 812.6 | 5666.3 |
| 3 | IM | IM | IN | 20.0 | 1011.1 | 7641.1 |
| 4 | IM | IM | IN | 20.0 | 888.3 | 8503.8 |
| 5 | IM | IM | | 20.0 | 980.2 | 2659.3 |

Analyzing neutralizing potency of nasal wash is a direct approach to demonstrate the superiority of sIgA-mediated anti-viral activity by IN booster. The results shown in Table 2 below demonstrate significantly enhanced nasal neutralizing activity induced by IM prime and IN boost vaccination regimen. Mucosal neutralizing potency from a single IN boost (Groups 3 and 4) is as efficacious as Group 2, which received two IN boosts. Nasal neutralizing activity was not found in IM-only mice (Group 5).

**Table 2: nasal wash neutralizing results**

| | Administration Route | | | Pseudovirus Neutralizing Assay |
|---|---|---|---|---|
| Groups | 1st | 2nd | 3rd | ID50 GMT |
| 1 | IM | IN | IN | 14.3 |
| 2 | IM | IN | IN | 54.9 |
| 3 | IM | IM | IN | 30 |
| 4 | IM | IM | IN | 34.6 |
| 5 | IM | IM | | <5 |

### Example 5: Hybrid vaccination against influenza viruses

Hybrid vaccination against influenza viruses, which also infect through mucosa, was further evaluated. The experimental materials, methods, and assays were substantially the same as those stated above, except for the antigens used in the vaccines. The antigens were obtained from the following influenza virus strains:
H1N1: A/Victoria/2570/2019 (H1N1)pdm09-like virus;
H3N2: A/Darwin/9/2021 (H3N2)-like virus;
B-Victoria: B/Austria/1359417/2021 (B/Victoria lineage)-like virus; and
B-Phuket: B/Phukef/3073/2013 (B/Yamagata lineage)-like virus.

The vaccine procedures of the hybrid vaccines are outlined in Table 3 below.

**Table 3: immunization scheme for hybrid vaccinations for influenza virus**

| Prime | Dosage of flu per strain (µg) | Week interval | Boost | Booster dosage of flu HA antigen per strain (µg) | Booster dosage of LTh(αK) to each strain (µg) |
|---|---|---|---|---|---|
| IM | 1 | | | | |
| IM | 1 | | IM | 1 | |
| IM | 1 | | IN | 1 | |
| IM | 1 | | IN | 1 | 3 |
| IM | 1 | | IM | 1 | |
| IM | 1 | | IN | 1 | I |
| IM | 1 | | IN | 1 | 3 |
| IM | 1 | 8 | IM | 1 | |
| IM | 1 | | IN | 1 | |
| IM | 1 | | IN | 1 | 3 |

Briefly, all animals received a primary shot of quadrivalent seasonal influenza vaccine containing one µg of hemagglutinin (HA) antigen per strain for a total of four strains via the intramuscular (IM) route. Three animals were sacrificed on Day 28 post-priming, and the serum, nasal wash, and bronchoalveolar lavage fluid (BALF) were collected and frozen before analysis. At two, four, or eight weeks post priming, animals were randomly selected and evenly divided into three regimens to receive IM booster, intranasal (IN) booster (HA only), or LTh(αK)-adjuvanted IN booster (HA+ LTh(αK)) for each interval (see Table 3 above). Four animals were included in each regimen for each interval; 28 days after the booster dose, animals were sacrificed and sera, nasal washes, and BALFs were collected for analysis. The results (Figs. 4A-4D) show that LTh(αK)-adjuvanted IN boosters increased the levels of the antigen-specific IgGs in serum. While LTh(αK) free boosters can elevate the levels of the antigen-specific IgGs, the elevation is less than that induced by LTh(αK)-adjuvanted boosters. Moreover, the enhanced levels of influenza antigen-specific IgGs were found through the 2-week to 8-week intervals. Moreover, the nasal washes and BALFs from animals in the hybrid vaccination were evaluated for the levels of secretory IgAs against influenza antigens. The results (Figs. 5A-5H) show that secretory IgAs were highly enhanced in the BALFs from animals treated with LTh(αK)-adjuvanted IN booster. In addition, similar to serum IgG levels, the enhanced levels of influenza antigen-specific IgAs were found through the 2-week to 8-week intervals. The ability of the IN booster to enhance IgG and sIgA levels for up to 8 weeks post priming indicates that the IN booster can be applied at a flexible time window in the hybrid vaccination scheme.

### Example 6: Short interval (1/2 hour) between IM prime and IN boost shows no boosting effect on the immunogenicity against vaccine antigens

. In this study, all animals received a primary shot as described in Example 5 above. As shown in Figs. 6A-6D, serum IgGs against influenza viruses H1N1, H3N2, B-Victoria and B-Phuket, respectively, were induced by IM prime. An IN booster dose was administrated 30 minutes after the prime dose. IgG levels were not elevated by the IN boosters, regardless of whether the booster contains the flu antigen, LTh(αK), or both. The results suggest that the interval between IM prime and IN boost for successfully boosting an immune response is unpredictable.

## Claims

1. An intranasal booster for use in a method for vaccinating a subject against a mucosal virus infection, comprising administering to the subject an immunologically effective amount of the intranasal booster, wherein the intranasal booster comprises detoxified *Escherichia coli* labile toxin (LT) and an antigen from the mucosal virus, and wherein the subject has been previously primed.

2. The intranasal booster for use of claim 1, wherein the mucosal virus is selected from coronavirus and influenza virus.

3. The intranasal booster for use of claim 2, wherein the coronavirus is SARS-CoV, MERS-CoV, or SARS-CoV-2, preferably SARS-CoV-2.

4. The intranasal booster for use of claim 3, wherein the antigen of the coronavirus is spike antigen.

5. The intranasal booster for use of claim 2, wherein the influenza virus is seasonal influenza virus.

6. The intranasal booster for use of any one of Claims 1 to 5, wherein the detoxified *Escherichia coli* labile toxin (LT) is LTh(αK).

7. The intranasal booster for use of any one of Claims 1 to 6, wherein the subject has been previously primed by intramuscular or intranasal vaccination.

8. The intranasal booster for use of any one of Claims 1 to 7, wherein the intranasal booster enhances one or more of serum IgG, serum IgA and mucosal IgA levels, or one or more of Th1, Th2 and Th17 cytokine production.

9. The intranasal booster for use of claim 8, wherein the cytokine is selected from IFN-y, IL-2, IL-5, IL-13, and IL-17.

10. An intranasal vaccine composition comprising an immunologically effective amount of a mucosal virus antigen adjuvanted with a detoxified *Escherichia coli* labile toxin (LT).

11. The vaccine composition of claim 10, wherein the mucosal virus is selected from coronavirus and influenza virus.

12. The vaccine composition of claim 11, wherein the coronavirus is SARS-CoV, MERS-CoV, or SARS-CoV-2, preferably SARS-CoV-2.

13. The vaccine composition of claim 12,wherein the antigen of the coronavirus is spike antigen

14. The vaccine composition of claim 11, wherein the influenza virus is seasonal influenza virus.

15. The vaccine composition of any one of Claims 10 to 14, wherein the detoxified *Escherichia coli* labile toxin (LT) is LTh(αK).
